# EUROPEAN PATENT SPECIFICATION

(11) **EP 2 280 884 B1**
(45) Date of publication and mention of the grant of the patent: **07.05.2014**
(21) Application number: 09731868.7
(22) Date of filing: 13.04.2009
(51) Int. Cl.: B65D 83/08, A61B 19/04

(54) **GLOVE PACKAGING HAVING ANTIMICROBIAL BARRIER**
HANDSCHUHVERPACKUNG MIT ANTIMIKROBIELLER SPERRE
EMBALLAGE POUR GANTS PRÉSENTANT UNE BARRIÈRE ANTIMICROBIENNE

(30) Priority: 18.04.2008 US 148448
(43) Date of publication of application: 09.02.2011
(73) Proprietor: Medline Industries, Inc., Mundelein, IL 60060 (US)
(72) Inventor: YAO, Min, Vernon Hills IL 60061 (US); AMDUR, Samuel, T., H., III, Libertyville IL 60048 (US)
(74) Representative: Grünecker, Kinkeldey, Stockmair & Schwanhäusser
(86) International application number: PCT/US2009/040370
(87) International publication number: WO 2009/129182

(56) References cited:
- WO-A1-2008/136721
- US-A- 3 997 703
- US-A- 4 675 347
- US-A- 5 357 636
- US-A- 5 501 323
- US-A1- 2002 043 537
- US-A1- 2004 091 678
- US-A1- 2005 150 788
- US-A1- 2008 054 011

## Description

The present invention relates to a packaging for gloves according to the preamble of independent claim 1 and a method for making a packaging for gloves. Such packaging for gloves can be taken from the prior art document US 5,501,323 A. More particularly, the present invention relates to packaging for gloves having an antimicrobial barrier for protecting the gloves from contamination from microorganisms and other undesirable materials or contaminants and methods for making the packaging.

Gloves are widely used as a protective measure and have become mandatory in many industries and nearly all medical and surgical settings. In particular, disposable gloves are required as a means for protecting medical and surgical staff from coming into contact with bodily fluids during surgical procedures, medical examinations, laboratory testing and other medical procedures. Disposable gloves have traditionally been made of rubber materials such as latex, thermoplastic materials such as vinyl, and other natural and synthetic materials.

Many gloves are provided in packaging having a cavity for holding the gloves. The packaging includes an opening for removing the gloves from the packaging. The opening is typically revealed by removing a perforated portion of the packaging to access the gloves. Once the perforated portion of the packaging is removed to reveal the opening, the gloves are exposed to the ambient environment. As the ambient environment may contain microorganisms, pathogens, small airborne particles of dust and debris and other air contaminants, the gloves contained in the packaging may be exposed to undesirable materials or contaminants that may contaminate the gloves while they are in the packaging.

Prior art document US 2002/0043537 A1 discloses a tissue dispenser having virucidal, bacterial, and/or germicidal properties is provided. The tissue dispenser includes a carton adapted to contain tissues having a plurality of walls and a carton opening. The carton can be made from numerous materials, including cardboard, paperboard, or plastic materials. Various anti-microbial agents known in the art can also be applied the exterior surface of the tissue dispenser to impart virucidal, bacterial, and/or germicidal properties thereto. The anti-microbial agent can comprise up to 100% of the surface area of the exterior surface of the dispenser, and in some embodiments, between about 10% to about 80%.

Prior art document US 5,501,323 A discloses a sealed dispenser including a cover having a first dispensing hole therein; a base having a second dispensing hole aligned with the first dispensing hole; and first and second flexible overlapping flaps mounted between the cover and base and covering the holes for forming a sealed barrier when the flaps are closed and permitting extraction of dispensable material when the flaps are opened.

The prior art is also disclosed in document WO 2008/136721 A1.

Thus, it is the object of the present invention to provide a packaging for gloves and a method for making a packaging for gloves that includes a barrier to protect gloves contained within the glove packaging from microorganisms, airborne particles and other materials or contaminants that may contaminate the gloves prior to removal from the packaging. According to the present invention said object is solved by a packaging for gloves having the features of independent claim 1 and a method for making a packaging for gloves having the features of independent claim 9. Preferred embodiments are laid down in the dependent claims.

Preferably, the barrier can also destroy any microorganisms, pathogens or other materials or contaminants that come in contact with the barrier to further reduce the possibility of contamination.

Accordingly, a packaging for gloves comprises a container having a cavity for holding the gloves. The container includes an opening for removing the gloves from the container. The packaging also comprises a barrier including an antimicrobial material positioned to cover at least a portion of the opening of the container.

In another embodiment of the present concepts, a method for making a packaging for gloves comprises providing a container having a cavity for holding the gloves. The container includes an opening for removing the gloves from the container. The method further comprises providing at least a first barrier having an antimicrobial material and attaching the first barrier to the container such that the barrier at least partially covers a portion of the opening of the container.

In yet another embodiment of the present concepts, a container for holding a plurality of gloves comprises a body portion, an opening and an antimicrobial film covering the opening. The antimicrobial film includes an aperture to facilitate removal of the gloves from the opening of the container.

The above explanation is not intended to represent each embodiment or every aspect of the present concepts. The detailed description and Figures will describe many of the embodiments and aspects of the present concepts.

### BRIEF DESCRIPTION OF THE DRAWINGS

The foregoing and other advantages of the invention will become apparent upon reading the following detailed description and upon reference to the drawings.
FIG. 1 is a perspective view of a glove packaging according to one embodiment of the present concepts.
FIG. 2 is a top view of the glove packaging illustrating an antimicrobial barrier.
FIG. 3 is a side view of a barrier including an antimicrobial material.
FIG. 4 is a side view of another embodiment of a barrier including an antimicrobial material.

While the invention is susceptible to various modifications and alternative forms, specific embodiments have been shown by way of example in the drawings and will be described in detail herein. It should be understood, however, that the invention is not intended to be limited to the particular forms disclosed. Rather, the invention is to cover all modifications, equivalents, and alternatives falling within the scope of the claims.

### DESCRIPTION OF ILLUSTRATIVE EMBODIMENTS

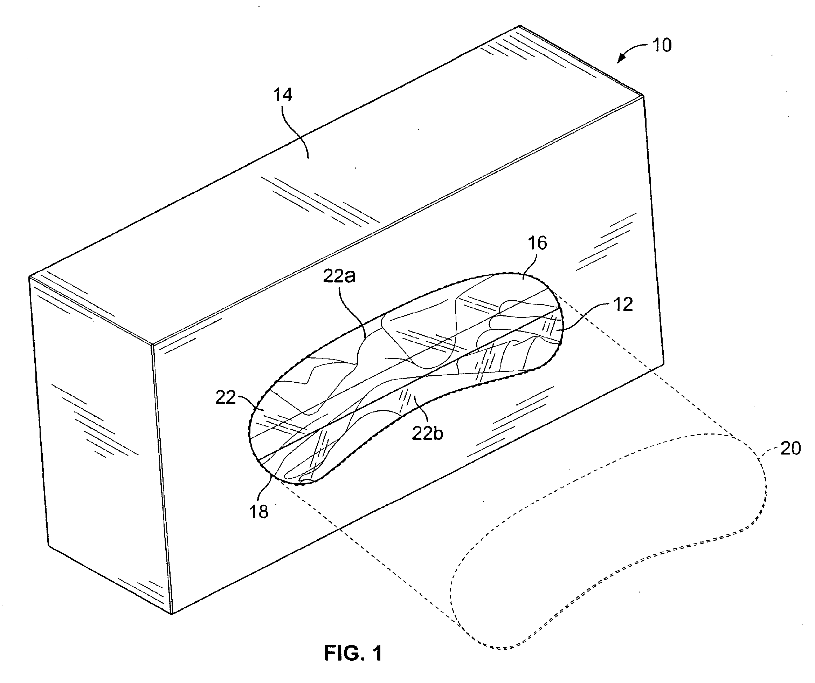

FIG. 1 illustrates a packaging 10 for gloves 12. The packaging 10 comprises a container 14 having a cavity 16 for holding the gloves 12. The container 14 includes an opening 18 for removing the gloves 12 from the container 14. The opening 18 may be in the form of different shapes, such as a circle, an oval, a square, a rectangle, or any variation of such shapes, such that a user may insert his or her hand through the opening 18 to remove one or more of the gloves 12. The opening 18 may initially be covered by a removable segment 20 that is initially formed as part of the container 14. The removable segment 20, which may be a perforated segment, is removable from the packaging 10 by a user once the packaging 10 is ready to be opened by tearing the removable segment 20 away from the packaging 10. The removable segment 20 is generally discarded after it is removed from the packaging 10. In addition to a perforated segment, the removable segment 20 may include an adhesive segment that is removable from the packaging 10.

The container 14 and removable segment 20 may be comprised of a variety of materials or combinations of materials, such as paper, plastic or fabric. The gloves 12 may include medical and/or surgical disposable gloves that are comprised of rubber materials such as latex, thermoplastic materials such as vinyl, and other natural and synthetic materials, such as nitrile, polyvinyl chloride, polyethylene, polyisoprene, neoprene, polychloriprene, etc. The gloves 12 may include other materials, such as antimicrobial coatings and/or coatings for protecting the skin that include aloe, chamomile, vitamin(s), or combinations thereof and other suitable ingredients that may provide skin care benefits, such as moisturizing and soothing dry, irritated skin. In addition to disposable gloves, it is contemplated that other types of gloves, i.e., non-medical or non-surgical gloves, could be used with the present concepts.

The packaging 10 may also comprise a barrier 22 that covers at least a portion of the opening 18 of the container 14. The barrier 22 may be a film, a piece of paper laminated with film or any type of flexible material that is suitable for providing or acting as a barrier. The barrier 22 inhibits or prevents microorganisms, pathogens, small airborne particles of dust and debris and other air contaminants from contacting and thus contaminating the gloves 12 prior to removal from the packaging 10. Thus, the barrier 22 helps to protect the gloves 12 from being exposed to undesirable materials or contaminants while the gloves 12 are in the packaging 10. Providing gloves 12 that are free from undesirable materials or contaminants reduces the risk that patients and healthcare workers will be exposed to such materials or contaminants and thereby reduces the opportunity to spread potentially harmful and infectious materials or contaminants. Furthermore, providing gloves 12 that are free from undesirable materials or contaminants also reduces or prevents cross-contamination that may occur between different patients.

In one embodiment, where the barrier 22 is a film, the film is a thin sheet of material, such as polypropylene, polystyrene, polyester, polyamide, polyvinylchloride, polyethylene (low density polyethylene, medium density polyethylene and/or high density polyethylene), polyvinylidene chloride, regenerated cellulose, cellulose acetate, and/or combinations thereof. The film material selected may be based on factors such as cost, shelf-life, barrier effectiveness, performance, etc. The film, including the paper-laminated film and any of the embodiments described herein, may have a thickness of less than about **0.25mm** (10 mils), particularly from about **0.025mm** (1 mil) to about **0.15mm** (6 mils). The thickness may be selected based on a variety of factors such as barrier effectiveness, cost, material(s) used, performance characteristics such as transparency and flexibility, etc. The film may be clear or colored. The film may also be printed or plain, and may be flat, patterned or embossed. Also, the film may be laminated with one or more other materials, in addition to paper, such as foil, vinyl or other materials. The film, including the paper-laminated film and any of the embodiments described herein, helps to prevent exposure of the inside of the container 14 to microorganisms, airborne particles and other materials or contaminants.

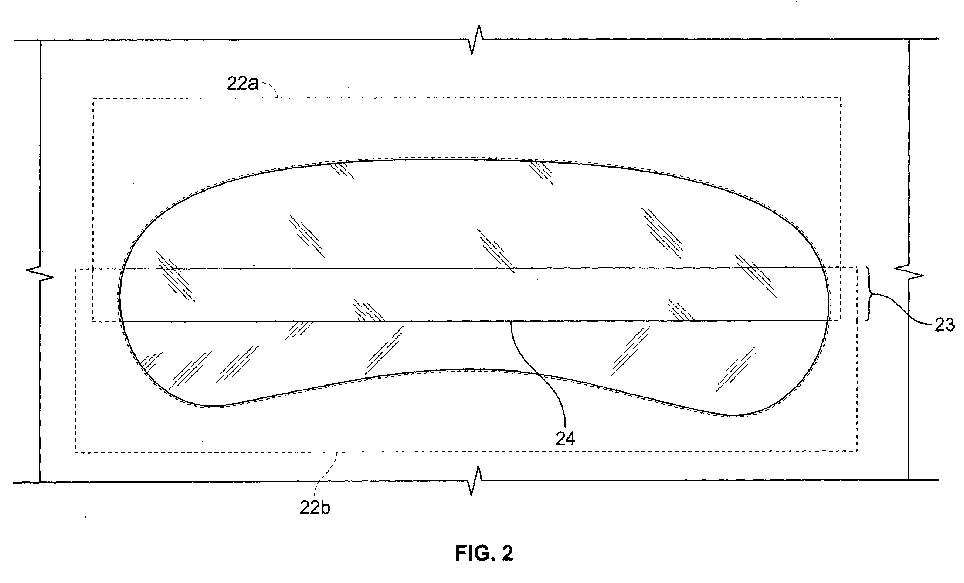

The barrier 22 may be a single sheet of film or may be multi-layered, as shown in FIGS. 1 and 2, as barriers 22a, 22b. The barriers 22, 22a, 22b may comprise the same type of or different materials. The barrier 22, 22a, 22b may also cover all or a portion of the top surface of the container 14, and may include an opening, aperture or slit in the middle of the barrier 22, 22a, 22b for removing the gloves 12. Having additional materials and/or additional layers may provide better protection than a single layer.

As shown in the embodiment of FIG. 2, the barriers 22a, 22b may include at least two overlapping films, including the paper-laminated film and any of the embodiments described herein, that are attached to the container 14. A first overlapping film or barrier 22a may cover a different portion of the opening 18 of the container 14 than a second overlapping film or barrier 22b. The barriers 22a, 22b may be positioned such that the barriers 22a, 22b are adjacent and overlap at an area corresponding approximately to the center of the opening 18, although it is contemplated that an overlapping portion 23 of the barriers 22a, 22b may be at other locations other than the center of the opening 18. Having the overlapping portions 23 at or near the center of the opening 18 may allow for larger areas for users to insert their hands to obtain one or more gloves 12, which may make removing the user's hand and glove easier. The overlapping portion 23 of the barriers 22a, 22b creates a slit 24 which allows a user to insert his or her hand through the slit 24 and remove a glove(s) 12 from the container 14. The barriers 22a, 22b may include a film, a piece of paper laminated with film or any type of flexible material that is suitable for providing or acting as a barrier.

Thus, one method for making the packaging 10 for gloves 12 includes providing a container 14 having a cavity 16 for holding the gloves 12 and an opening 18 for removing the gloves 12 from the container 14. Once a barrier, such as barrier 22, 22a, 22b, is provided which includes the antimicrobial material, a suitable method for attaching the barrier 22, 22a, 22b can be used to cover at least a portion of the opening 18 of the container 14. In some embodiments, the barrier 22, 22a, 22b includes overlapping films or barriers that cover the opening 18 of the container 14 and form the slit 24 for removal of the gloves 12 from the container 14.

In the embodiments shown in FIGS. 1 and 2, the barriers 22, 22a, 22b are attached to an inside surface of the top portion of the container 14. The barriers 22, 22a, 22b may be attached to the container 14 via adhesive material, glue, heat bonding, mechanical bonding, such as staples, and other suitable modes of attachment. The barriers 22, 22a, 22b may be attached along one or more peripheries, or other suitable area, of the top of the container 14. The barriers 22, 22a, 22b themselves may be made according to various methods, including extrusion, extrusion coating, co-extrusion or calendaring, or other suitable methods for making films. Extrusion, for example, is one of the most common and inexpensive methods for making a film.

The barrier 22, 22a, 22b of the embodiments described herein may include an antimicrobial material(s). The antimicrobial material(s) may include, but is/are not limited to, silver-based antimicrobial materials, copper-based antimicrobial materials, chlorhexidene gluconate, benzalkonium chloride, monoquaternary and polyquaternary ammonium salt-based antimicrobial materials (including covalent bonded quaternary ammonium salt (QAS)), biguanide-based antimicrobials such as polyhexamethylene biguanide (PHMB), triclosan, zinc pyrithione, isothiazolinone-based antimicrobials, 10,10'-oxybisphenoxarsine-based (OPBA) antimicrobials, peptide-based antimicrobials, natural antimicrobials such as hops extract, honey and chitosan-based antimicrobials, and any combinations thereof. The antimicrobial material may be selected based on a variety of factors, such as an efficacy requirement (percent of reduction), time to kill, antimicrobial spectrum, i.e., how broadly the antimicrobial material can kill bacteria, or other viruses, mold, fungi, etc. The amount of antimicrobial material used may depend on the specific antimicrobial material used, as different antimicrobial materials will require different levels for effectiveness. Thus, the amount of antimicrobial material needed will vary, but each antimicrobial material will have an antimicrobially effective level.

The antimicrobial material may be added to the barrier 22, 22a, 22b according to different methods that include, but are not limited to, spraying, coating, mixing with a polymer before extrusion, or other methods suitable to result in an application or addition of the antimicrobial material to the barrier 22, 22a, 22b. The particular method chosen may depend on the type of manufacturing process being used to make the barrier 22, 22a, 22b, the end use of the product, cost and other relevant factors. In some embodiments, spraying may be the most cost effective method. In other embodiments, the antimicrobial material may be added to the barrier 22, 22a, 22b by mixing the antimicrobial material with the barrier material before extrusion. Mixing may be advantageous as it does not require additional steps in the manufacturing process. All of these methods provide for the antimicrobial material to be included on the surface of the barrier 22, 22a, 22b, distributed within the barrier 22, 22a, 22b, or both.

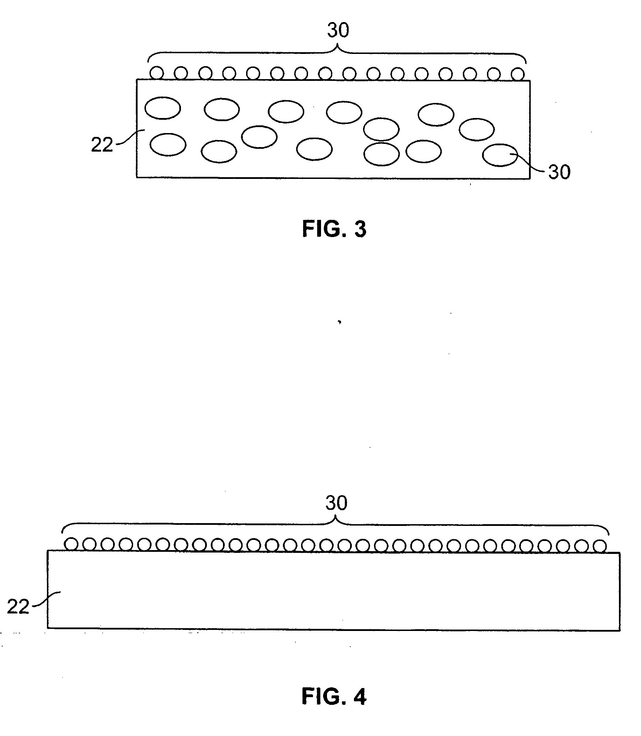

As shown in FIG. 3, the antimicrobial material 30 may be uniformly added to the surface of and incorporated within the barrier 22, 22a, 22b such that once a microorganism comes in contact with the barrier 22, 22a, 22b, it comes into contact with the antimicrobial material 30 and is killed. While it is possible to add the antimicrobial material 30 to only a portion of the barrier 22, 22a, 22b, such as coating the surface of the barrier 22, 22a, 22b with the antimicrobial material 30 as shown in FIG. 4, it is generally more economical and effective to treat the whole barrier 22, 22a, 22b (FIG. 3). In some embodiments, the antimicrobial material 30 distributed within the barrier 22, 22a, **22b** may migrate to the surface of the barrier 22, 22a, 22b once the antimicrobial material 30 on the surface is consumed.

Thus, the antimicrobial material 30 that is added to the barrier 22, 22a, 22b destroys the microorganisms and pathogens that may come in contact with the barrier 22, 22a, 22b and thus reduces and/or eliminates the amount of microorganisms that may be deposited on the disposable gloves 12 housed within the container 14. The antimicrobial material 30 in combination with the barrier 22, 22a, 22b creates a contaminant-free zone on the packaging 10 that assists in reducing or eliminating patients and healthcare workers' exposure to potentially infectious and harmful microorganisms and contaminants. Additionally, the barrier 22, 22a, 22b prevents or reduces the occurrence of airborne particles and other materials or contaminants that may contaminate the gloves prior to removal from the packaging.

## Claims

1. A packaging for gloves (12), the packaging comprising:
a container (14) having a cavity (16) configured to hold the gloves (12), the container (14) having an opening (18) through which the gloves (12) are removable from the container (14);
a first barrier (22a) positioned to cover at least a first portion of the opening (18) of the container (14); and
a second barrier (22b) positioned to cover at least a second portion of the opening (18) different from the first portion, wherein the first barrier (22a) is adjacent to and at least partially overlaps the second barrier (22b), the first and second barriers (22a,22b) forming a slit (24) through which the gloves (12) are removable, **characterized in that** the first barrier (22a) includes a first antimicrobial material, and the second barrier (22b) has a second antimicrobial material the same as or different from the first antimicrobial material of the first barrier (22a).

2. The packaging of claim 1, wherein the second antimicrobial material of the second barrier (22b) is different from the first antimicrobial material of the first barrier (22a).

3. The packaging of claims 1 or 2, wherein the first and second barriers (22a,22b) comprise at least two overlapping films.

4. The packaging of claims 1 to 3, wherein the first and second barriers (22a,22b) each comprises polypropylene, polystyrene, polyester, polyamide, polyvinylchloride, polyethylene, polyvinylidene chloride, regenerated cellulose, or cellulose acetate or any combination thereof.

5. The packaging of claims 1 to 4, wherein the first and second antimicrobial materials (30) each comprises silver-based antimicrobial materials (30), copper-based antimicrobial materials (30), chlorhexidene gluconate, benzalkonium chloride, monoquaternary and polyquaternary ammonium salt-based antimicrobial materials (30), biguanide-based antimicrobials such as polyhexamethylene biguanide, triclosan, zinc pyrithione, isothiazolinone-based antimicrobials, 10,10'-oxybisphenoxarsine-based antimicrobials, peptide-based antimicrobials, natural antimicrobials such as hops extract, or honey and chitosan-based antimicrobials, or any combination thereof.

6. The packaging of claims 1 to 5, wherein the first and second antimicrobial materials (30) are each added to the barriers by at least one of spraying, coating, or mixing with the barrier before extrusion.

7. The packaging of claims 1 to 6, wherein the first and second barriers (22a,22b) each has a thickness of less than about 0.25 mm (10 mils).

8. The packaging of claim 7, wherein the first and second barriers (22a,22b) each has a thickness of from about 0.025 mm (1 mil) to about 0.15 mm (6 mils).

9. A method for making a packaging for gloves (12) according to claim 1, the method comprising:
providing a container (14) having a cavity (16) configured to hold the gloves (12), the container (14) having an opening (18) through which the gloves (12) are removable from the container (14);
providing a first barrier (22a) having a first antimicrobial material;
attaching the first barrier (22a) to the container (14) such that the first barrier (22a) covers at least a first portion of the opening (18) of the container (14);
providing a second barrier (22b) having a second antimicrobial material the same as or different from the first antimicrobial material of the first barrier (22a); and
attaching the second barrier (22b) to the container (14) such that the second barrier (22b) covers at least a second portion of the opening (18) different from the first portion,
wherein the first barrier (22a) is adjacent to and at least partially overlaps the second barrier (22b), the first and second barriers (22a,22b) forming a slit (24) through which the gloves (12) are removable.

10. The method of claim 9, wherein the second antimicrobial material of the second barrier (22b) is different from the first antimicrobial material of the first barrier (22a).

11. The method of claims 9 or 10, wherein the first and second barriers (22a,22b) are attached to an inside surface of the container (14) via an adhesive material.

12. The method of claims 9 to 11, wherein the first barrier (22a) and the second barrier (22b) comprise at least two overlapping films.

13. The method of claims 9 to 12, wherein the first barrier (22a) and the second barrier (22b) each has a thickness of less than about 0.25 mm (10 mils).

## Patentansprüche

1. Eine Verpackung für Handschuhe (12), wobei die Verpackung umfasst:
einen Behälter (14) mit einem Hohlraum (16), der dazu konfiguriert ist, die Handschuhe (12) zu enthalten, wobei der Behälter (14) eine Öffnung (18) aufweist, durch welche die Handschuhe (12) aus dem Behälter (14) entnehmbar sind;
eine erste Barriere (22a), die so angeordnet ist, dass sie zumindest einen ersten Teil der Öffnung (18) des Behälters (14) verdeckt; und
eine zweite Barriere (22b), die so angeordnet ist, dass sie zumindest einen zweiten Teil der Öffnung (18) verdeckt, der sich von dem ersten Teil unterscheidet, wobei die erste Barriere (22a) benachbart zu der zweiten Barriere (22b) angeordnet ist und diese zumindest teilweise überlappt, die erste und zweite Barriere (22a, 22b) einen Schlitz (24) bilden, durch den die Handschuhe (12) entnehmbar sind, **dadurch gekennzeichnet, dass** die erste Barriere (22a) ein erstes antimikrobielles Material beinhaltet und die zweite Barriere (22b) ein zweites antimikrobielles Material aufweist, das dasselbe oder ein anderes Material als das erste antimikrobielle Material der ersten Barriere (22a) ist.

2. Die Verpackung nach Anspruch 1, wobei das zweite antimikrobielle Material der zweiten Barriere (22b) sich von dem ersten antimikrobiellen Material der ersten Barriere (22a) unterscheidet.

3. Die Verpackung nach Anspruch 1 oder 2, wobei die erste und zweite Barriere (22a, 22b) mindestens zwei überlappende Folien umfassen.

4. Die Verpackung nach Anspruch 1 bis 3, wobei die erste und zweite Barriere (22a, 22b) jeweils Polypropylen, Polystyrol, Polyester, Polyamid, Polyvinylchlorid, Polyethylen, Polyvinylidenchlorid, regenerierte Zellulose oder Zellulose-Acetat oder eine Kombination daraus umfasst.

5. DieVerpackung nach Anspruch 1 bis 4, wobei das erste und zweite antimikrobielle Material (30) jeweils Silber-basierte antimikrobielle Materialien (30), Kupfer-basierte antimikrobielle Materialien (30), Chlorhexidingluconat, Benzalkoniumchlorid, monoquaternäre und polyquaternäre Ammoniumsalz-basierte antimikrobielle Materialien (30), Biguanid-basierte antimikrobielle Stoffe wie Polyhexamethylenbiguanidhydrochlorid, Triclosan, Zinkpyrithion, lsothiazolinon-basierte antimikrobielle Stoffe, 10,10'-Oxybisphenoxarsin-basierte antimikrobielle Stoffe, Peptid-basierte antimikrobielle Stoffe, natürliche antimikrobielle Stoffe wie Hopfenextrakt oder Honig- und Chitosanbasierte antimikrobielle Stoffe oder eine Kombination daraus umfassen.

6. Die Verpackung nach Anspruch 1 bis 5, wobei das erste und zweite antimikrobielle Material (30) jeweils durch mindestens eine Methode aus Sprühen, Beschichten oder Mischen mit der Barriere vor dem Extrudieren zu den Barrieren hinzugefügt werden.

7. Die Verpackung nach Anspruch 1 bis 6, wobei die erste und zweite Barriere (22a, 22b) jeweils eine Dicke von weniger als etwa 0,25 mm (10 mils) aufweisen.

8. Die Verpackung nach Anspruch 7, wobei die erste und zweite Barriere (22a, 22b) jeweils eine Dicke von weniger als etwa 0,025 mm (1 mil) bis etwa 0,15 mm (6 mils) aufweisen.

9. Ein Verfahren zur Herstellung einer Verpackung für Handschuhe (12) nach Anspruch 1, wobei das Verfahren umfasst:
Bereitstellen eines Behälters (14) mit einem Hohlraum (16), der dazu konfiguriert ist, die Handschuhe (12) zu enthalten, wobei der Behälter (14) eine Öffnung (18) aufweist, durch welche die Handschuhe (12) aus dem Behälter (14) entnehmbar sind;
Bereitstellen einer ersten Barriere (22a) mit einem ersten antimikrobiellen Material;
Befestigen der ersten Barriere (22a) an dem Behälter (14) so dass die erste Barriere (22a) zumindest einen ersten Teil der Öffnung (18) des Behälters (14) verdeckt;
Bereitstellen einer zweiten Barriere (22b9 mit einem zweiten antimikrobiellen Material, das dasselbe oder ein anderes als das erste antimikrobielle Material der ersten Barriere (22a) ist; und
Befestigen der zweiten Barriere (22b) an dem Behälter (14) so dass die zweite Barriere (22a) zumindest einen zweiten Teil der Öffnung (18) des Behälters (14) verdeckt, der sich von dem ersten Teil unterscheidet;
wobei die erste Barriere (22a) benachbart zu der zweiten Barriere (22b) angeordnet ist und diese zumindest teilweise überlappt, die erste und zweite Barriere (22a, 22b) einen Schlitz (24) bilden, durch den die Handschuhe (12) entnehmbar sind.

10. Das Verfahren nach Anspruch 9, wobei das zweite antimikrobielle Material der zweiten Barriere (22b) sich von dem ersten antimikrobiellen Material der ersten Barriere (22a) unterscheidet.

11. Das Verfahren nach Anspruch 9 oder 10, wobei die erste und zweite Barriere (22a, 22b) mithilfe eines Klebstoffes an einer Innenfläche des Behälters (14) befestigt sind.

12. Das Verfahren nach Anspruch 9 bis 11, wobei die erste Barriere (22a) und die zweite Barriere (22b) mindestens zwei überlappende Folien umfassen.

13. Das Verfahren nach Anspruch 9 bis 12, wobei die erste Barriere (22a) und die zweite Barriere (22b) jeweils eine Dicke von weniger als etwa 0,25 mm (10 mils) aufweisen.

## Revendications

1. Emballage pour gants (12), l'emballage comprenant :
un contenant (14) présentant une cavité (16) conçue pour contenir les gants (12), le contenant (14) présentant une ouverture (18) par laquelle les gants (12) peuvent être sortis du contenant (14) ;
une première barrière (22a) placée de manière à recouvrir au moins une première partie de l'ouverture (18) du contenant (14) ; et
une seconde barrière (22b) placée de manière à recouvrir au moins une seconde partie de l'ouverture (18) différente de la première partie, où la première barrière (22a) est adjacente à et chevauche au moins partiellement la seconde barrière (22b), les première et seconde barrières (22a, 22b) formant une fente (24) par laquelle les gants (12) peuvent être sortis, **caractérisé en ce que** la première barrière (22a) comprend un premier matériau antimicrobien, et la seconde barrière (22b) a un second matériau antimicrobien identique au premier matériau antimicrobien de la première barrière (22a) ou différent de ce premier matériau.

2. Emballage de la revendication 1, dans lequel le second matériau antimicrobien de la seconde barrière (22b) est différent du premier matériau antimicrobien de la première barrière (22a).

3. Emballage des revendications 1 ou 2, dans lequel les première et seconde barrières (22a,22b) comprennent au moins deux pellicules qui se chevauchent.

4. Emballage des revendications 1 à 3, dans lequel les première et seconde barrières (22a,22b) comprennent chacune du polypropylène, du polystyrène, du polyester, du polyamide, du polychlorure de vinyle, du polyéthylène, du polychlorure de vinylidène, de la cellulose régénérée, ou de l'acétate de cellulose ou toute combinaison des précédents.

5. Emballage des revendications 1 à 4, dans lequel les premier et second matériaux antimicrobiens (30) comprennent chacun des matériaux antimicrobiens (30) à base d'argent, des matériaux antimicrobiens (30) à base de cuivre, du gluconate de chlorhexidine, du chlorure de benzalkonium, des matériaux antimicrobiens (30) à base de sel d'ammonium monoquaternaire et polyquaternaire, des antimicrobiens à base de biguanide tels que le polyhexaméthylène biguanide, du triclosan, de la pyrithione de zinc, des antimicrobiens à base d'isothiazolinone, des antimicrobiens à base de 10,10'-oxybisphénoxarsine, des antimicrobiens à base de peptide, des antimicrobiens naturels tels que l'extrait de houblon, ou le miel et des antimicrobiens à base de chitosane, ou toute combinaison des précédents.

6. Emballage des revendications 1 à 5, dans lequel les premier et second matériaux antimicrobiens (30) sont chacun ajoutés aux barrières par au moins une méthode parmi la pulvérisation, le revêtement, ou le mélange avec la barrière avant extrusion.

7. Emballage des revendications 1 à 6, dans lequel les première et seconde barrières (22a,22b) ont chacune une épaisseur inférieure à environ 0,25 mm (10 millièmes de pouce).

8. Emballage de la revendication 7, dans lequel les première et seconde barrières (22a,22b) ont chacune une épaisseur d'environ 0,025 mm (1 millième de pouce) à environ 0,15 mm (6 millièmes de pouce).

9. Procédé de fabrication d'un emballage pour gants (12) selon la revendication 1, le procédé comprenant :
la fourniture d'un contenant (14) présentant une cavité (16) conçue pour contenir les gants (12), le contenant (14) présentant une ouverture (18) par laquelle les gants (12) peuvent être sortis du contenant (14) ;
la fourniture d'une première barrière (22a) ayant un premier matériau antimicrobien ;
la fixation de la première barrière (22a) au contenant (14) de façon à ce que la première barrière (22a) recouvre au moins une première partie de l'ouverture (18) du contenant (14) ;
la fourniture d'une seconde barrière (22b) ayant un second matériau antimicrobien identique au premier matériau antimicrobien de la première barrière (22a) ou différent de ce premier matériau ; et
la fixation de la seconde barrière (22b) au contenant (14) de façon à ce que la seconde barrière (22b) recouvre au moins une seconde partie de l'ouverture (18) différente de la première partie, où la première barrière (22a) est adjacente à et chevauche au moins partiellement la seconde barrière (22b), les première et seconde barrières (22a,22b) formant une fente (24) par laquelle les gants (12) peuvent être sortis.

10. Procédé de la revendication 9, dans lequel le second matériau antimicrobien de la seconde barrière (22b) est différent du premier matériau antimicrobien de la première barrière (22a).

11. Procédé des revendications 9 ou 10, dans lequel les première et seconde barrières (22a,22b) sont fixées à une surface intérieure du contenant (14) par l'intermédiaire d'un matériau adhésif.

12. Procédé des revendications 9 à 11, dans lequel la première barrière (22a) et la seconde barrière (22b) comprennent au moins deux pellicules qui se chevauchent.

13. Procédé des revendications 9 à 12, dans lequel la première barrière (22a) et la seconde barrière (22b) ont chacune une épaisseur inférieure à environ 0,25 mm (10 millièmes de pouce).
